# EUROPEAN PATENT SPECIFICATION

(11) **EP 1 632 229 B1**
(45) Date of publication and mention of the grant of the patent: **01.04.2009**
(21) Application number: 04019003.5
(22) Date of filing: 11.08.2004
(51) Int. Cl.: A61K 31/4439, A61K 31/46, A61K 31/14, A61P 13/02

(54) **Medicaments for the treatment of urinary tract disorders comprising anticholinergic agents**
Anticholinergika enthaltende Arzneimittel zur Behandlung von Krankheiten der ableitenden Harnwege
Médicaments pour le traitement des maladies des voies urinaires contenant des anticholinergiques

(43) Date of publication of application: 08.03.2006
(73) Proprietor: Boehringer Ingelheim Pharma GmbH & Co. KG, 55216 Ingelheim am Rhein (DE)
(72) Inventor: Pieper, Michael P., 88400 Biberach (DE)
(74) Representative: Hammann, Heinz

(56) References cited:
- WO-A-01/17480
- WO-A-02/32898
- WO-A-02/32899
- WO-A-03/002059
- WO-A-03/064417
- WO-A-03/064418
- WO-A-03/064419
- WO-A-20/04064789
- WO-A-20/04064821
- US-A- 5 998 430
- US-B1- 6 482 837
- SCHELFHOUT V.J. ET AL.: "Activity of LAS 34273, a new Long Acting Anticholinergic Agent, in COPD Patients"[Online] 19 May 2004 (2004-05-19), XP002298563 Retrieved from the Internet: URL:http://www.abstracts2view.com/atsall/s earch.php?query=Schelfhout&where[]=authors &search=do> [retrieved on 2004-09-28]

## Description

The present invention relates to a the use of one or more, preferably one long acting anticholinergic for the preparation of a medicament for the treatment of urinary tract disorders selected from among urolithiasis, urinary tract cysts and urinary tract polyps.

### Background of the invention:

Urinary tract disorders as for instance urinary incontinence (UI) caused by e.g. unstable detrusor /detrusor instability causes significant morbidity in sufferers and a great financial expense to health care providers. The condition is associated with symptoms of increased urinary frequency, urgency and urge incontinence. The embarrassing nature of these symptoms means patients are often reluctant to seek medical help and as a consequence there is under-reporting. Prevalence of UI is up to 30-40 % in male and female patients.

The present invention targets these receptors by administration of long acting anticholinergic agents via various routes of administration to attain prolonged maintenance of bladder control in otherwise incontinent patients. Furthermore, it is the object of the present invention to provide for alternative, advantageous methods for the treatment of urinary tract disorders, as for instance urinary incontinence.

US 6 482 837 B1 discloses that antimuscarinic compounds in general, and the class of glycolate esters of heterocyclic amino alcohols in particular (as the compounds of present formulas **1a** and **1c-1g**) can be used in the treatment of incontinence. This document further suggests the use of any quaternarized antimuscarinic agent in order to prevent penetration of the BBB and so to prevent central side effects.

US 5 998 430 A discloses the use of trospium chloride for the treatment of bladder dysfunction like incontinence.

WO 2004/064789 A discloses the use of tiotropium bromide for the treatment of bladder diseases, in particular incontinence.

WO 01/17480 A discloses that glycopyrrolate (=glycopyrronium bromide) is an anticholinergic agent which can be used in the treatment of bladder diseases like incontinence.

### Description of the invention

The present invention relates to the use of one or more, preferably one long acting anticholinergic **1** for the preparation of a medicament for the treatment of urinary tract disorders selected from among urolithiasis, urinary tract cysts and urinary tract polyps, wherein the long acting anticholinergic **1** is selected from among tiotropium bromide, glycopyrronium bromide and trospium chloride.

The term "long acting anticholinergic" defines anticholinergic agents that show a long duration of action. These long acting anticholinergics show a pharmacokinetic profile that allows administration once or twice, preferably once per day.

In a preferred embodiment the invention relates to the use of one or more, preferably one long acting anticholinergic **1** for the preparation of a medicament for the treatment of the urinary tract disorders mentioned hereinbefore, wherein the long acting anticholinergic **1** is selected from among specific tiotropium salts, glycopyrronium salts and trospium salts. In the above-mentioned salts the cations tiotropium, glycopyrronium and trospium are the pharmacologically active components. Within the scope of the present patent application, an explicit reference to the above cations is indicated by the use of the number **1'**. Any reference to the aforementioned salts **1** naturally also includes a reference to the ingredients **1'** (tiotropium, glycopyrronium or trospium). Glycopyrronium salts are preferably used in their enantiomerically pure form. From the various diastereomers known for glycopyrronium salts the R,R-g-lycopyrronium salts are of outstanding importance. By the salts **1** which may be used within the scope of the present invention are meant the compounds which contain, in addition to tiotropium and glycopyrronium as counter-ion (anion), bromide, in addition to trospium as counter-ion chloride. Of particular importance according to the invention are salts **1** selected from among enantiomerically pure glycopyrronium bromide and tiotropium bromide. In the use according to the invention tiotropium bromide is particularly preferred. The aforementioned salts may be optionally present in form of their solvates or hydrates, preferably in form of their hydrates. If tiotropium bromide is used it is preferably present in form of its crystalline tiotropium bromide monohydrate as disclosed in WO 02/30928. In case tiotropium bromide is used in anhydrous form, it is preferably present in form of the crystalline tiotropium bromide anhydrate disclosed in WO 03/000265.

Optionally the long acting anticholinergic agents mentioned hereinbefore possess chiral carbon centers. In this case the pharmaceutical compositions according to the invention may contain the long acting anticholinergic agents in form of their enantiomers, mixtures of enantiomers or racemats. Preferably chiral long acting anticholinergics are present in form of one of their pure enantiomers.

Within the use of the present invention, the long acting anticholinergic **1** may be administered by oral, parenteral (e.g., intramuscular,intraperitoneal, intravenous or subcutaneous injection, or implant), buccal, nasal, vaginal, rectal, sublingual, topical (e.a.. ocular eyedrop) or inhalative routes of administration and may be formulated in suitable dosage unit formulations containing conventional non-toxic pharmaceutically acceptable carriers, adjuvants and vehicles appropriate for each route of administration.

The pharmaceutical compositions for the administration of the long acting anticholinergics **1** of this invention may conveniently be presented in dosage unit form and may be prepared by any of the methods well known in the art of pharmacy. All methods include the step of bringing the active ingredient into association with the carrier which is constituted of one or more accessory ingredients. In general, the pharmaceutical compositions are prepared by uniformly and intimately bringing the active ingredients into association with a liquid carrier or a finely divided solid carrier or both, and then, if necessary, shaping the product into the desired dosage form. In the pharmaceutical compositions the active compounds are included in an amount sufficient to produce the desired pharmacologic effect.

The pharmaceutical compositions containing the active ingredients **1** that are suitable for oral administration may be in the form of discrete units such as hard or soft capsules, tablets, troches or lozenges, each containing a predetermined amount of the active ingredients; in the form of a dispersible powder or granules; in the form of a solution or a suspension in an aqueous liquid or non-aqueous liquid; in the form of syrups or elixirs; or in the form of an oil-in-water emulsion or a water-in-oil emulsion.

Dosage forms intended for oral use may be prepared according to any method known to the art for the manufacture of pharmaceutical formulations and such compositions.

The excipients used may be for example, (a) inert diluents such as mannitol, sorbitol, calcium carbonate, pregelatinized starch, lactose, calcium phosphate or sodium phosphate; (b) granulating and disintegrating agents, such as povidone, copovidone, hydroxypropylmethylcellulose, corn starch, alginic acid, crospovidone, sodiumstarchglycolate, croscarmellose, or polacrilin potassium ; (c) binding agents such as microcrystalline cellulose or acacia ; and (d) lubricating agents such as magnesium stearate, stearic acid, fumaric acid or talc.

In some cases, formulations for oral use may be in the form of hardgelatin or HPMC capsules wherein the active ingredient **1** is mixed with an inert solid diluent, for example pregelatinized starch, calcium carbonate, calcium phosphate or kaolin, or dispensed via a pellet formulation. They may also be in the form of soft gelatin capsules wherein the active ingredient is mixed with water or an oil medium, for example peanut oil, liquid paraffin, medium chain triglycerides or olive oil.

The tablets, capsules or pellets may be uncoated or they may be coated by known techniques to delay disintegration and absorption in the gastrointestinal tract and thereby provide a delayed action or sustained action over a longer period. For example, a time delay material such as celluloseacetate phtalate or hydroxypropylcellulose acetate succinate or sustained release material such as ethylcellulose or ammoniomethacrylate copolymer (type B) may be employed.

Liquid dosage forms for oral administration include pharmaceutically acceptable emulsions, solutions, suspensions, syrups, and elixirs containing inert diluents commonly used in the art, such as water. Besides such inert diluents, compositions can also include adjuvants, such as wetting agents, emulsifying and suspending agents, and sweetening, flavoring, perfuming and preserving agents.

Aqueous suspensions normally contain the active ingredient **1** in admixture with excipients suitable for the manufacture of aqueous suspensions. Such excipients may be (a) suspending agents such as hydroxy ethylcellulose, sodium carboxymethylcellulose, methylcellulose, hydroxypropylmethylcellulose, sodium alginate, polyvinylpyrrolidone, gum tragacanth and gum acacia; (b) dispersing or wetting agents which may be (b.1) a naturally-occurring phosphatide such as lecithin, (b.2) a condensation product of an alkylene oxide with a fatty acid, for example, polyoxyethylene stearate, (b.3) a condensation product of ethylene oxide with a long chain aliphatic alcohol, for example heptadecaethyleneoxycetanol, (b.4) a condensation product of ethylene oxide with a partial ester derived from a fatty acid and a hexitol such as polyoxyethylene sorbitol monooleate, or (b.5) a condensation product of ethylene oxide with a partial ester derived from a fatty acid and a hexitol anhydride, for example polyoxyethylene sorbitan monooleate.

The aqueous suspensions may also contain one or more preservatives, for example, ethyl or n-propyl p-hydroxybenzoate; one or more coloring agents; one or more flavoring agents; and one or more sweetening agents, such as sucrose or saccharin.

Oily suspensions may be formulated by suspending the active ingredients **1** in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents and flavoring agents may be added to provide a palatable oral preparation. These compositions may be prepared by the addition of an antioxidant such as ascorbic acid.

Dispersible powders and granules are suitable for the preparation of an aqueous suspension. They provide the active ingredients **1** in admixture with a dispersing or wetting agent, a suspending agent and one or more preservatives. Suitable dispersing or wetting agents and suspending agents are exemplified by those already mentioned above. Additional excipients, for example, those sweetening, flavoring and coloring agents described above may also be present.

The pharmaceutical compositions of the invention may also be in the form of oil-in-water emulsions. The oily phase may be a vegetable oil such as olive oil or arachis oils, or a mineral oil such as liquid paraffin or a mixture thereof.

Suitable emulsifying agents may be (a) naturally-occurring gums such as gum acacia and gum tragacanth, (b) naturally-occurring phosphatides such as soybean and lecithin, (c) esters or partial esters derived from fatty acids and hexitol anhydrides, for example, sorbitan monooleate, (d) condensation products of said partial esters with ethylene oxide, for example polyoxyethylene sorbitan monooleate. The emulsions may also contain sweetening and flavoring agents.

Syrups and elixirs may be formulated with sweetening agents, for example, glycerol, propylene glycol, sorbitol or sucrose. Such formulations may also contain a preservative and flavoring and coloring agents.

The pharmaceutical compositions containing **1** may be in the form of a sterile injectable aqueous or oleagenous suspension or solution. The suspension may be formulated according to known methods using those suitable dispersing or wetting agents and suspending agents which have been mentioned above. The sterile injectable preparation may also be a sterile injectable solution or suspension in a non toxic parenterally-acceptable diluent or solvent, for example as a solution in 1,3-butane-diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

Preparations according to this invention containing **1** for parenteral administration include sterile aqueous or non-aqueous solutions, suspension, or emulsions.

Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions which can be reconstituted in sterile water, or some other sterile injectable medium immediately before use. The combination of this invention may also be administered in the form of suppositories for rectal administration. This composition can be prepared by mixing the drugs with a suitable nonirritating excipient which is solid at ordinary temperatures but liquid at the rectal temperature and will therefore melt in the rectum to release the drug. Such materials are cocoa butter, hard fat, and polyethylene glycols. Compositions for buccal, nasal or sublingual administration are also prepared with standard excipients well known in the art.

For topical administration the compositions according to this invention containing **1** may be formulated in liquid or semi-liquid preparations such as liniments, lotions, applications; oil-in-water or water-in-oil emulsions such as creams, ointments, jellies or pastes, including tooth-pastes; or solutions or suspensions such as drops, and the like.

The dosage of the active ingredients in the compositions of this invention may be varied. However, it is necessary that the amount of the active ingredients **1** be such that a suitable dosage form is obtained. The selected dosage and the dosage form depend upon the desired therapeutic effect, on the route of administration and on the duration of the treatment. Dosage ranges in the combination are approximately one tenth to one times the clinically effective ranges required to induce the desired therapeutic effect, respectively when the compounds are used singly.

Within the use according to the invention the long acting anticholinergics **1** may also be administered by inhalation. Inhalable preparations according to the invention include inhalable powders, propellant-containing metered dose aerosols or propellant-free inhalable solutions. Inhalable powders according to the invention containing the active substances may consist of the active substances on their own or of a mixture of the active substances with physiologically acceptable excipients. Within the scope of the present invention, the term carrier may optionally be used instead of the term excipient. Within the scope of the present invention, the term propellant-free inhalable solutions also includes concentrates or sterile inhalable solutions ready for use.

Inhalable powders may contain **1** either alone or in admixture with suitable physiologically acceptable excipients. If the active substance **1** is present in admixture with physiologically acceptable excipients, the following physiologically acceptable excipients may be used to prepare these inhalable powders according to the invention: monosaccharides (e.g. glucose or arabinose), disaccharides (e.g. lactose, saccharose, maltose, trehalose), oligo- and polysaccharides (e.g. dextran), polyalcohols (e.g. sorbitol, mannitol, xylitol), cyclodextrines (e.g. α-cyclodextrine, β-cyclodextrine, χ-cyclodextrine, methyl-β-cyclodextrine, hydroxypropyl-β-cyclodextrine), salts (e.g. sodium chloride, calcium carbonate) or mixtures of these excipients with one another. Preferably, mono- or disaccharides are used, while the use of lactose, trehalose or glucose is preferred, particularly, but not exclusively, in the form of their hydrates.

Inhalable powders preferably contain excipients with a maximum average particle size of up to 250µm, preferably between 10 and 150µm, most preferably between 15 and 80µm. It may sometimes seem appropriate to add finer excipient fractions with an average particle size of 1 to 9µm to the excipient mentioned above. These finer excipients are also selected from the group of possible excipients listed hereinbefore. Finally, in order to prepare the inhalable powders according to the invention, micronised active substance, preferably with an average particle size of 0.5 to 10µm, more preferably from 1 to 6µm, is added to the excipient mixture. Processes for producing the inhalable powders according to the invention by grinding and micronising and by finally mixing the ingredients together are known from the prior art.

The inhalable powders according to the invention may be administered using inhalers known from the prior art. Inhalable powders according to the invention which contain one or more physiologically acceptable excipients in addition to **1** may be administered, for example, by means of inhalers which deliver a single dose from a supply using a measuring chamber as described in US 4570630, or by other means as described in DE 36 25 685. The inhalable powders according to the invention which contain **1** optionally in conjunction with a physiologically acceptable excipient may be administered, for example, using the inhaler known by the name Turbuhaler^{®} or using inhalers as disclosed for example in EP 237507. Preferably, the inhalable powders according to the invention which contain physiologically acceptable excipient in addition to **1** are packed into capsules (to produce so-called inhalettes) which are used in inhalers as described, for example, in WO 94/28958.

Inhalation aerosols containing propellant gas may contain substance **1** dissolved in the propellant gas or in dispersed form. The propellant gases which may be used to prepare the inhalation aerosols according to the invention are known from the prior art. Suitable propellant gases are selected from among hydrocarbons such as n-propane, n-butane or isobutane and halohydrocarbons such as fluorinated derivatives of methane, ethane, propane, butane, cyclopropane or cyclobutane. The propellant gases mentioned above may be used on their own or in mixtures thereof. Particularly preferred propellant gases are halogenated alkane derivatives selected from TG11, TG12, TG134a (1,1,1,2-tetrafluoroethane) and TG227 (1,1,1,2,3,3,3-heptafluoropropane) and mixtures thereof, of which the propellant gases TG134a, TG227 and mixtures thereof are preferred.

The propellant-driven inhalation aerosols according to the invention may also contain other ingredients such as co-solvents, stabilisers, surfactants, antioxidants, lubricants and pH adjusters. All these ingredients are known in the art.

The inhalation aerosols containing propellant gas according to the invention may contain up to 5 wt.-% of active substance **1**. Aerosols according to the invention contain, for example, 0.002 to 5 wt.-%, 0.01 to 3 wt.-%, 0.015 to 2 wt.-%, 0.1 to 2 wt.-%, 0.5 to 2 wt.-% or 0.5 to 1 wt.-% of active substance **1**.

If the active substances **1** are present in dispersed form, the particles of active substance preferably have an average particle size of up to 10µm, preferably from 0.1 to 6µm, more preferably from 1 to 5µm.

The propellant-driven inhalation aerosols according to the invention mentioned above may be administered using inhalers known in the art (MDIs = metered dose inhalers). Suitable cartridges and methods of filling these cartridges with the inhalable aerosols containing propellant gas according to the invention are known from the prior art.

For example, in the use according to the invention tiotropium may be administered for instance in such amounts that each individual dose contains preferably 0.1 - 100µg. For example, and without restricting the scope of the invention thereto, 2.5µg, 5µg, 10µg, 18µg, 20µg, 36µg or 40µg of tiotropium (calculation based on cation) may be administered per single dose.

For example, and without restricting the scope of the invention thereto, in the use according to the invention glycopyrronium, preferably R,R-glycopyrronium may be administered for instance in such amounts that each individual dose contains preferably 1 - 300µg. For example, and without restricting the scope of the invention thereto, 25µg, 35µg, 45µg, 55µg, 65µg, 75µg, 85µg, 95µg, 105µg, 115µg, 125µg, 135µg, 145µg, 155µg, 165µg, 175µg, 185µg or 195µg of glycopyrronium (calculation based on cation) may be administered per single dose. Preferably the aforementioned doses are administered once, twice or three times per day, preferably twice or three times per day.

Within the scope of the present invention, any reference to the compounds **1'** is to be regarded as a reference to the pharmacologically active cations contained in the salts **1**. These are the cations tiotropium, glycopyrronium or trospium.

The Examples which follow serve to illustrate the present invention in more detail.

### Examples of Formulations

The following examples of formulations, which may be obtained analogously to methods known in the art, serve to illustrate the present invention more fully without restricting it to the contents of these examples.

Tablets:
1)

| **Ingredients** | **mg per suppository** |
|---|---|
| tiotropium bromide | 3.3 |
| dibasic calcium phosphate, anhydrous | 33 |
| maize starch dried | 30 |
| starch, soluble | 2 |
| silica colloidal | 4 |
| tartaric acid granule | 0.5 |
| stearic acid powder | 0.5 |
| povidone K25 | 0.505 |
| sucrose refined | 41.194 |
| talc 405 | 23.671 |
| acacia powdered | 2.761 |
| titanium dioxide | 1.802 |
| macrogel 6000 | 0.04 |
| carnauba wax | 0.018 |
| beeswax white | 0.009 |

Ampoules:
1)

| **Ingredients** | **content per ampoule** |
|---|---|
| tiotropium bromide | 0.17 mg |
| NaCl | 6 mg |
| water purified | ad 1 ml |

Solution for injection:
1)

| **Ingredients** | **content** |
|---|---|
| tiotropium bromide | 0.17 mg |
| methyl-4-hydroxybenzoate | 18 mg |
| Propyl-4-hydroxybenzoate | 2 mg |
| NaCl | 60 mg |
| water purified | ad 10 ml |

Suppositories:
1)

| **Ingredients** | **mg per suppository** |
|---|---|
| tiotropium bromide | 3.3 |
| water purified | 10 |
| hard fat | 1626.7 |
| Total | 1640 |

## Claims

1. Use of one or more, preferably one long acting anticholinergic **1** for the preparation of a medicament for the treatment of urinary tract disorders selected from among urolithiasis, urinary tract cysts and urinary tract polyps,
wherein the long acting anticholinergic **1** is selected from among tiotropium bromide, glycopyrronium bromide and trospium chloride.

2. Use according to claim 1 wherein the long acting anticholinergic **1** is used for the preparation of a medicament suitable for oral, parenteral, buccal, nasal, vaginal, rectal, sublingual, topical or inhalative administration.

3. Long acting anticholinergic **1** selected from among tiotropium bromide, glycopyrronium bromide and trospium chloride for use in the treatment of urinary tract disorders selected from among urolithiasis, urinary tract cysts and urinary tract polyps.

## Patentansprüche

1. Verwendung von einem oder mehreren, bevorzugt seinem langwirkenden Anticholinergikum **1** zur Herstellung eines Arzneimittels zur Behandlung von Störungen der Harnwege, ausgewählt aus Urolithiasis, Harnwegzysten und Harnwegpolypen, wobei das langwirkende Anticholinergikum **1** ausgewählt ist aus Tiotropiumbromid, Glycopyrroniumbromid und Trospiumchlorid.

2. Verwendung nach Anspruch 1, wobei das langwirkende Anticholinergikum **1** zur Herstellung eines Arzneimittels verwendet wird, das zur oralen, parenteralen, buccalen, nasalen, vaginalen, rektalen, sublingualen, topischen oder inhalativen Verabreichung geeignet ist.

3. Langwirkendes Anticholinergikum **1**, ausgewählt aus Tiotropiumbromid, Glycopyrroniumbromid und Trospiumchlorid, zur Verwendung bei der Behandlung von Störungen der Harnwege, ausgewählt aus Urolithiasis, Harnwegzysten und Harnwegpolypen.

## Revendications

1. Utilisation d'un ou plusieurs, de préférence d'un anticholinergique à longue durée d'action **1** pour la préparation d'un médicament pour le traitement des troubles des voies urinaires choisis parmi l'urolithiase, les kystes des voies urinaires et les polypes des voies urinaires, où l'anticholinergique à longue durée d'action **1** est choisi parmi le bromure de tiotropium, le bromure de glycopyrronium et le chlorure de trospium.

2. Utilisation selon la revendication 1 où l'anticholinergique à longue durée d'action **1** est utilisé pour la préparation d'un médicament approprié à l'administration orale, parentérale, buccale, nasale, vaginale, rectale, sublinguale, topique ou par inhalation.

3. Anticholinergique à longue durée d'action **1** choisi parmi le bromure de tiotropium, le bromure de glycopyrronium et le chlorure de trospium destiné à être utilisé dans le traitement des troubles des voies urinaires choisis parmi l'urolithiase, les kystes des voies urinaires et les polypes des voies urinaires.
